# EUROPÄISCHE PATENTSCHRIFT

(11) **EP 2 996 616 B1**
(45) Veröffentlichungstag und Bekanntmachung des Hinweises auf die Patenterteilung: **25.03.2020**
(21) Anmeldenummer: 14721310.2
(22) Anmeldetag: 28.04.2014
(51) Int. Cl.: A61B 34/30, B25J 15/04

(54) **HALTEEINRICHTUNG FÜR EIN CHIRURGISCHES INSTRUMENT**
HOLDING DEVICE FOR A SURGICAL INSTRUMENT
SYSTÈME D'ARRÊT POUR UN INSTRUMENT CHIRURGICAL

(30) Priorität: 16.05.2013 DE 102013209122
(43) Veröffentlichungstag der Anmeldung: 23.03.2016
(73) Patentinhaber: Deutsches Zentrum für Luft- und Raumfahrt e.V., 51147 Köln (DE)
(72) Erfinder: LANTERMANN, Sophie, 80939 München (DE); SEIBOLD, Ulrich, Burnaby British Columbia V3N 4Z4 (CA); HAGN, Ulrich, 81543 München (DE); STÄBLER, Thomas, 72070 Tübingen (DE)
(74) Vertreter: dompatent von Kreisler Selting Werner - Partnerschaft von Patent- und Rechtsanwälten mbB
(86) Internationale Anmeldenummer: PCT/EP2014/058599
(87) Internationale Veröffentlichungsnummer: WO 2014/183980

(56) Entgegenhaltungen:
- WO-A1-2011/040813
- WO-A1-2012/071408
- WO-A2-2009/061915
- US-A1- 2011 277 775

## Beschreibung

Die Erfindung betrifft eine Halteeinrichtung für ein chirurgisches Instrument, insbesondere für ein in der minimal-invasiven Chirurgie eingesetztes Instrument.

In der minimal-invasiven Chirurgie werden chirurgische Instrumente durch sogenannte Trokare in das Körperinnere des Patienten eingeführt. Die Trokare halten kleine Einschnitte in der Haut des Patienten offen, sodass lange, üblicherweise stabförmige Instrumente in den Situs eingeführt werden können. Während der Operation werden unterschiedliche Instrumente benötigt, sodass ein Instrumentenwechsel erforderlich ist. Dies ist insbesondere erforderlich, da nur eine möglichst geringe Anzahl an Trokaren vorgesehen ist. Bei der robotischen Chirurgie wird das chirurgische Instrument von einem Roboterarm gehalten und geführt. Ein entsprechender, insbesondere mehrere über Gelenke miteinander verbundene Elemente aufweisender Haltearm trägt einen Instrumententräger, der an seinem distalen Ende das chirurgische Instrument trägt. Über bspw. in dem Instrumententräger angeordnete Zugseile, Wellen und dergleichen wird das chirurgische Instrument betätigt. Die Betätigung erfolgt über eine Antriebseinrichtung.

Aus Guthart, GS und Salisbury, JK: The Intuitive Telesurgery System: Overview and Application, Proceedings of ICRA 2000 ist es bekannt, die Antriebseinrichtung innerhalb des Haltearms anzuordnen. Zum Wechseln des chitriebseinrichtung innerhalb des Haltearms anzuordnen. Zum Wechseln des chirurgischen Instruments wird der Instrumententräger vom Haltearm getrennt und sodann entweder durch einen neuen Instrumententräger mit neuem chirurgischem Instrument ersetzt oder das chirurgische Instrument an dem Instrumententräger wird ausgetauscht. Hierbei erfolgt ein Verschwenken des Instrumententrägers und somit ein Verschwenken des Instruments im Patienten. Ggf. ist das chirurgische Instrument auch unmittelbar mit dem Haltearm verbunden, sodass ein Instrumententräger entfällt. Dieses Verschwenken kann zu Verletzungen führen. Desweiteren muss über Zwischenelemente ein Verbinden der Antriebseinrichtung mit dem Instrumententräger realisiert werden.
Desweiteren ist es aus Hagn, U. et al: DLR MiroSurge: a versatile system for research in endoscopic telesurgery, International journal of computer assisted radiology and surgery, 5, 2, Seiten 183-193, 2010, Springer bekannt, die Antriebseinheit zusammen mit dem Instrumententräger bzw. zusammen mit dem chirurgischen Instrument zu wechseln. Dies hat den Vorteil, dass eine feste Verbindung mit dem Instrumententräger möglich ist und ferner stets die auf den entsprechenden Instrumententräger und das chirurgische Instrument abgestimmte Antriebseinrichtung vorgesehen sein kann. Andererseits weist dies den Nachteil auf, dass die Komponenten teurer sind, da mit jedem Instrumententräger eine Antriebseinrichtung verbunden ist. Hierbei ist es zwar möglich, die Antriebseinrichtung von dem Instrumententräger zu trennen und sodann die Antriebseinrichtung mit dem neuen Instrumententräger zu verbinden, dies erfordert jedoch eine zweihändige und zeitaufwändige Manipulation. Das chirurgische Instrument kann einstückig mit dem Instrumententräger ausgebildet sein bzw. der Instrumententräger kann entfallen, sodass das chirurgische Instrument unmittelbar mit der Antriebseinheit verbunden ist.
Aus WO 2011/040813 ist ein Operationsroboter mit einer automatisierten Instrumentenwechselvorrichtung bekannt. Weiterer relevanter Stand der Technik wird in den Dokumenten WO2012/071408, US2011/277775 und WO2009/061915 offenbart.

Aufgabe der Erfindung ist es, eine Halteeinrichtung für chirurgische Instrumente zu schaffen, bei der ein einfaches Auswechseln von Instrumententrägern möglich ist. Ferner ist es Aufgabe der Erfindung, ein vereinfachtes Verfahren zum Auswechseln eines chirurgischen Instruments an einer Halteeinrichtung zu schaffen.

Die Lösung der Aufgabe erfolgt erfindungsgemäß hinsichtlich der Vorrichtung durch die Merkmale des Anspruchs 1 und hinsichtlich des Verfahrens durch die Merkmale des Anspruchs 10.

Die erfindungsgemäße Halteeinrichtung für chirurgische Instrumente, die insbesondere für den Einsatz in der minimal-invasiven Chirurgie geeignet ist, weist einen Haltearm, wie einen insbesondere mehrere Gelenke aufweisenden Roboterarm auf. Von dem Haltearm wird der Instrumententräger getragen. An dem distalen Ende des Instrumententrägers ist ein chirurgisches Instrument angeordnet. Hierbei kann das chirurgische Instrument auswechselbar mit dem Instrumententräger oder einstückig mit dem Instrumententräger ausgebildet sein. Hierbei erstreckt sich das chirurgische Instrument über einen größeren Bereich und ist nicht nur am distalen Ende des Instrumententrägers angeordnet. Das chirurgische Instrument selbst kann somit sehr klein ausgebildet sein und mit dem distalen Ende des Instrumententrägers auswechselbar verbunden sein. Andererseits ist eine einstückige Ausgestaltung möglich, sodass der Instrumententräger mit dem chirurgischen Instrument einstückig ausgebildet ist bzw. der Instrumententräger entfällt, sodass das chirurgische Instrument unmittelbar mit dem Haltearm verbunden ist. Der Instrumententräger kann durch einen Trokar in das Patienteninnere zur Durchführung einer Operation eingeführt werden. Desweiteren ist eine Antriebseinrichtung zum Betätigen des chirurgischen Instruments mit dem Instrumententräger insbesondere lösbar verbunden. Erfindungsgemäß ist die Antriebseinrichtung über eine Halteeinrichtung mit dem Haltearm verbunden. Hierdurch ist es möglich, dass die Antriebseinrichtung aus einer Antriebsposition in eine Auswechselposition überführt werden kann. In der Antriebsposition ist die Antriebseinrichtung mit dem Instrumententräger und somit mit dem chirurgischen Instrument zum Betätigen des chirurgischen Instruments verbunden. In der Auswechselposition ist die Antriebseinrichtung vom Instrumententräger derart entkoppelt, dass ein Auswechseln des Instrumententrägers möglich ist. Die Halteeinrichtung dient somit einerseits zum Halten bzw. Fixieren der Antriebseinrichtung an dem Haltearm und andererseits zum Herstellen einer lösbaren Verbindung mit dem Instrumententräger bzw. dem chirurgischen Instrument.

Zum Auswechseln des Instrumententrägers kann die Antriebseinrichtung, ohne vollständig vom Haltearm gelöst werden zu müssen, in eine Auswechselposition überführt werden. In dieser Position, in der die Antriebseinrichtung entkoppelt ist, ist es sodann möglich auf einfache Weise den Instrumententräger zu wechseln. Nach dem Wechseln des Instrumententrägers, d.h. nachdem der neue Instrumententräger wieder mit dem Haltearm verbunden ist, wird die Antriebseinrichtung mithilfe der Halteeinrichtung aus der Auswechselposition wieder in die Antriebsposition zurückgeführt, sodass auf einfache Weise die Antriebseinrichtung wieder mit dem Instrumententräger gekoppelt ist.

Ggf. können der Instrumententräger und der Haltearm auch einstückig ausgebildet sein.

Die Halteeinrichtung weist ein Schwenkelement auf. Mithilfe des Schwenkelements ist es möglich, die Antriebseinrichtung zwischen der Antriebs- und der Auswechselposition zu verschwenken bzw. zu verdrehen. Hierbei ist es bevorzugt, dass automatisch beim Verschwenken bzw. Verdrehen ein Koppeln bzw. Entkoppeln erfolgt.

Bei einer weiteren bevorzugten Ausführungsform weist die Halteeinrichtung ein Schiebeelement auf, sodass die Antriebseinrichtung zwischen den beiden Positionen verschiebbar ist, wobei wiederrum vorzugsweise automatisch ein Koppeln bzw. Entkoppeln erfolgt.

Das Schiebelement kann vorzugsweise eine Führungsschiene aufweisen, um beim Überführen der Antriebseinrichtung von der einen Position in die andere Position eine definierte Führung zu gewährleisten. Die Führungsschiene kann hierbei bspw. eine gekrümmte Führungsbahn aufweisen, in der mit der Antriebseinrichtung verbundene Führungszapfen gleiten können.

Selbstverständlich ist auch eine Kombination eines oder mehrerer Schwenkelemente mit einem oder mehreren Schiebelementen möglich.

Bei einer bevorzugten Weiterbildung der Erfindung weist die Halteeinrichtung mindestens ein Fixierelement zum Fixieren der Antriebseinrichtung in der Antriebsposition und/oder der Auswechselposition auf. Ggf. ist je Position ein Fixierelement vorgesehen. Bei dem Fixierelement kann es sich um ein Rastelement, ein bspw. durch einen Magneten ausgebildetes Halteelement oder dergleichen handeln. Ist bspw. ein Fixierelement zum Fixieren der Antriebseinrichtung in der Auswechselposition vorgesehen, ist hierdurch sichergestellt, dass beim Auswechseln des Instrumententrägers keine ungewollte Positionsänderung der Antriebseinrichtung erfolgt. Diese könnte zu Erschütterungen und somit Verletzungen des Patienten etc. führen. Ein Fixierelement, durch das die Antriebseinrichtung in der Antriebsposition gehalten ist, weist den Vorteil auf, dass während der Operation ein ungewolltes Verändern der Position der Antriebseinrichtung vermieden ist.

Bei einer weiteren bevorzugten Ausführungsform weist die Halteeinrichtung mindestens ein Dämpfungselement auf. Durch das Dämpfungselement wird die Bewegung der Antriebseinrichtung bei Veränderung der Position der Antriebseinrichtung gedämpft. Vorzugsweise ist zumindest ein Dämpfungselement vorgesehen, durch das die Bewegung der Antriebseinrichtung in die Antriebsposition und/oder die Auswechselposition gedämpft wird. Ggf. kann je Position ein gesondertes Dämpfungselement vorgesehen sein. Durch Vorsehen mindestens eines Dämpfungselements werden beim Verändern der Position der Antriebseinrichtung eine Erschütterung und die dadurch hervorgerufenen Nachteile vermieden.

Bei einer besonders bevorzugten Ausführungsform ist der Instrumententräger in der Antriebsposition unmittelbar mit der Antriebseinrichtung, insbesondere über Steckkontakte verbunden. Eine Verbindung über ein Zwischenelement, das bei einem entfernteren Anordnen der Antriebseinrichtung am Roboterarm erforderlich wäre, ist daher nicht erforderlich. Hierdurch können insbesondere Ungenauigkeiten in der Übertragung von Bewegungen vermieden werden.

Bei einer weiteren bevorzugten Ausführungsform ist es möglich, mit der Halteeinrichtung eine Zusatz-Antriebseinrichtung zu verbinden. Hierdurch können auf einfache Weise auch ggf. erforderliche zusätzliche Antriebe bei besonderen Instrumenten vorgesehen werden. Bspw. sind bei manchen Instrumenten stärkere Antriebe erforderlich.

Ein wesentliches Merkmal der vorstehenden Erfindungen besteht somit darin, dass die Antriebseinheit vom Instrumententräger teilbar ist. Es handelt sich nicht um fest miteinander verbundene Bauteile. In die Antriebseinheit kann hierbei neben dem Motor auch Steuerelektronik integriert sein. Der Instrumententräger ist, da es sich insbesondere um einen Instrumententräger für die minimal-invasive Chirurgie handelt, lang und dünn ausgebildet und wird in den Patienten eingeführt. Während der Operation ist es erforderlich chirurgische Instrumente, die von dem Instrumententräger getragen werden, zu wechseln. Beispielsweise ist der Wechsel von einem Nadelhalter auf eine Schere oder Gewebegreifer oder dergleichen erforderlich. Erfindungsgemäß ist es auf Grund der Möglichkeit, die Antriebseinrichtung in eine Auswechselposition zu überführen, möglich, dass die Antriebseinrichtung mit dem Haltearm verbunden bleibt und somit der unabhängig von der Antriebseinrichtung ausgestaltete Instrumententräger gewechselt werden kann. Dies stellt ein erhebliche Kostenersparnis dar, da nicht jeder Instrumententräger mit einer Antriebseinrichtung versehen werden muss, sondern eine Antriebseinrichtung für eine Vielzahl von Instrumententrägern genutzt werden kann. Ferner ist es auf Grund der erfindungsgemäßen Verbindung zwischen der Antriebseinheit und dem Haltearm vorteilhaft, dass die Antriebseinheit nicht abgelegt werden muss und somit auch keine Kontaminationsgefahr entsteht. Mit der erfindungsgemäßen Halteeinrichtung ist ein schnelles und sicheres Wechseln des Instrumententrägers möglich.

Ein wesentlicher Aspekt der Erfindung besteht somit darin, dass die Antriebseinrichtung einerseits in vorteilhafter Weise einfach und sicher mit dem Instrumententräger gekoppelt werden kann und andererseits mit dem Haltearm derart verbunden ist, dass auf vereinfachte Weise ein Überführen der Antriebseinrichtung in die Auswechseleinrichtung möglich ist.

Ferner betrifft die Erfindung ein Verfahren zum Auswechseln eines chirurgischen Instruments, insbesondere eines chirurgischen Instruments für die minimal-invasive Chirurgie an einer Halteeinrichtung. Die Halteeinrichtung weist zumindest einen, von einem Haltearm getragenen Instrumententräger auf, der zum Betätigen eines mit dem Instrumententräger verbundenen chirurgischen Instruments mit einer Antriebseinheit verbunden ist. Die Halteeinrichtung ist vorzugsweise wie vorstehend erläutert vorteilhaft weitergebildet. Gemäß dem erfindungsgemäßen Verfahren wird die Antriebseinheit aus einer Antriebsposition in eine Auswechselposition überführt. Dies kann durch Verschwenken, Verschieben und dergleichen erfolgen, wie insbesondere vorstehend anhand der Halteeinrichtung beschrieben ist. In der Auswechselposition erfolgt ein Auswechseln des Instrumententrägers, wobei die Antriebseinheit in der Auswechselposition mit dem Haltearm verbunden bleibt.

Vorzugsweise erfolgt ein Trennen des Instrumententrägers von einer Antriebseinrichtung beispielsweise vor dem Überführen der Antriebseinrichtung in die Auswechselposition. Bevorzugt ist es, dass diese Trennung während bzw. durch das Überführen selbst erfolgt. Es ist insbesondere bevorzugt, dass die elektrischen oder mechanischen Kontakte zwischen der Antriebseinrichtung und der Instrumententräger durch das Überführen der Antriebseinrichtung in die Auswechselposition getrennt werden. Hierdurch ist eine einfache und schnelle Handhabung und Auswechselung des Instrumententrägers ermöglicht.

Das erfindungsgemäße Verfahren ist insbesondere wie vorstehend anhand der Halteeinrichtung beschrieben vorteilhaft weitergebildet.

Nachfolgend wird die Erfindung anhand bevorzugter Ausführungsformen unter Bezugnahme auf die anliegenden Zeichnungen näher erläutert.

Es zeigen:
- Fig. 1a: eine schematische Darstellung einer ersten Ausführungsform in einer Antriebsposition,
- Fig. 1b: eine schematische Darstellung der in Fig. 1a dargestellten ersten Ausführungsform in der Auswechselposition,
- Fig. 2a: eine schematische Darstellung einer zweiten Ausführungsform in einer Antriebsposition,
- Fig. 2b: eine schematische Darstellung der in Fig. 2a dargestellten zweiten Ausführungsform in der Auswechselposition,
- Fig. 3a: eine schematische Darstellung einer dritten Ausführungsform in einer Antriebsposition,
- Fig. 3b: eine schematische Darstellung der in Fig. 3a dargestellten dritten Ausführungsform in der Auswechselposition,
- Fig. 4a: eine schematische Darstellung einer vierten Ausführungsform in einer Antriebsposition,
- Fig. 4b: die in Fig. 4a dargestellte Ausführungsform in Seitenansicht in Richtung des Pfeils B in Fig. 4a,
- Fig. 4c: eine schematische Darstellung der in den Figuren 4a und 4b dargestellten Ausführungsform in einer Auswechselposition,
- Fig. 5a: eine schematische Darstellung einer nicht zur Erfindung gehörenden Ausführungsform in einer Antriebsposition und
- Fig. 5b: eine schematische Darstellung der in Fig. 5a dargestellten Ausführungsform in der Auswechselposition.

In den dargestellten bevorzugten Ausführungsformen der Erfindung sind ähnliche und identische Bauteile mit denselben Bezugszeichen gekennzeichnet.

Die Halteeinrichtungen weisen einen im dargestellten Ausführungsbeispiel als Roboterarm ausgebildeten Haltearm 10 auf. Der Haltearm 10 weist mehrere über Gelenke 14 miteinander verbundene Elemente 12 auf. Mit dem letzten der an seinem distalen Ende ein chirurgisches Instrument 20 trägt. Die Aufnahme des Instrumentenhalters 18 erfolgt durch eine in dem Aufnahmeelement 16 vorgesehene Einstecköffnung 22. Ferner weist der Instrumententräger 18 ein Kopfelement 24 auf, das insbesondere über Steckkontakte mit einer Antriebseinrichtung 26 verbunden ist. Die Antriebseinrichtung 26 ist über eine Halteeinrichtung 28 mit dem Haltearm 10 bzw. dem Aufnahmeelement 16 des Haltearms 10 verbunden.

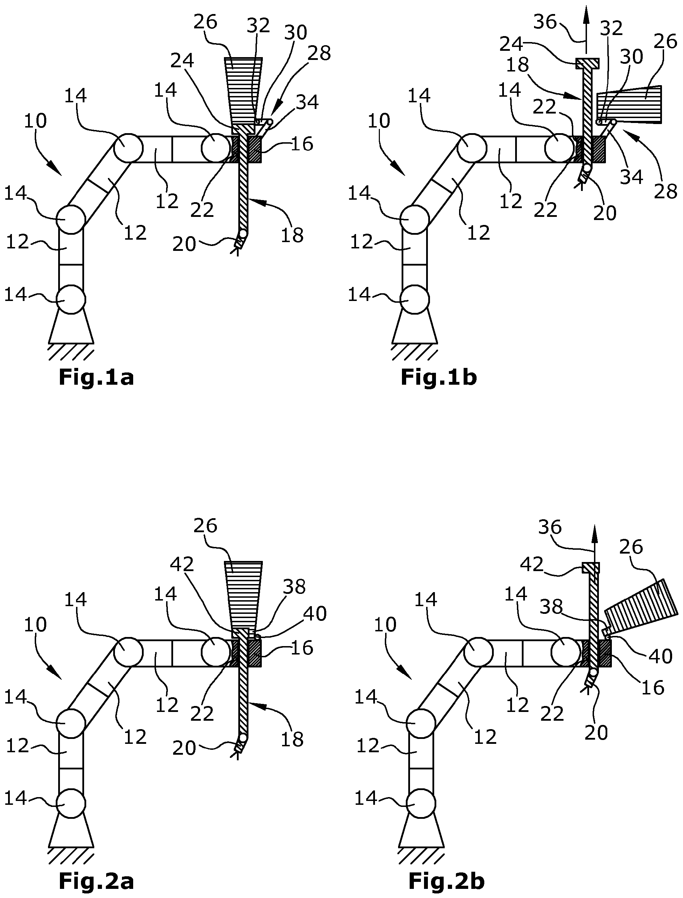

In der ersten dargestellten Ausführungsform weist das Halteelement 28 ein Schwenkelement 30 auf. Das Schwenkelement 30 ist im dargestellten Ausführungsbeispiel über ein Gelenk 32 mit der Antriebseinrichtung 26 verbunden. Desweiteren weist die Halteeinrichtung 28 in dem in den Figuren 1a und 1b dargestellten Ausführungsbeispiel einen Abstandshalter 34 auf. Der Abstandshalter 34 dient dazu, dass die Antriebseinrichtung 26 in der Antriebsposition (Fig. 1a) in einer Lage angeordnet ist, in der der Kopf 24 in Fig. 1a unterhalb der Antriebseinrichtung 26 angeordnet und sicher mit der Antriebeinrichtung 26 verbunden ist.

Zum Auswechseln des Instrumententrägers 18 wird die Antriebseinrichtung 26 in die in Fig. 1b dargestellte Position verschwenkt. Es ist sodann möglich, den Instrumententräger 18 in Richtung des Pfeils 36 in Fig. 1b nach oben aus dem Aufnahmeelement 16 des Haltearms 10 herauszuziehen. Nach erfolgtem Wechseln des Instruments 20 am distalen Ende des Instrumententrägers 18 oder nach vollständigem Austausch des Instrumententrägers kann dieser wieder in die Öffnung 22 eingeführt und die Antriebseinrichtung 26 aus der Auswechselposition (Fig. 1b) in die Antriebsposition (Fig. 1a) zurückverschwenkt werden.

Es ist möglich, nicht dargestellte Dämpfungselemente vorzusehen, um beim Verschwenken der Antriebseinrichtung 26 in die unterschiedlichen Positionen Erschütterungen im Haltearm zu vermeiden oder zumindest zu dämpfen. Desweiteren können Fixierelemente vorgesehen sein, durch die die Antriebseinrichtung vorzugsweise in beiden Positionen fixiert ist.

Bei der in Fig. 2 dargestellten Ausführungsform weist die Antriebseinrichtung 26 bzw. das Gehäuse der Antriebseinrichtung 26 einen Ansatz 38 auf. Dieser weist in Richtung des Aufnahmeelements 16. Die Halteeinrichtung ist in dieser Ausführungsform als Gelenk 40 ausgebildet, das zwischen dem Ansatz 38 und dem Aufnahmeelement 16 angeordnet ist. Desweiteren ist ein Kopf 42 des Instrumententrägers 18 derart ausgestaltet, dass in der Antriebsposition (Fig. 2a) der Ansatz 38 neben dem Kopf 42 angeordnet ist. Hierdurch ist eine kompaktere Bauweise möglich. Ferner kann die Halteeinrichtung einfacher ausgestaltet sein.

Die Antriebseinrichtung 26 ist ähnlich wie in dem ersten Ausführungsbeispiel aufgrund des vorgesehenen Gelenks 40 in eine Auswechselposition verschwenkbar (Fig. 2b), sodass der Instrumententräger 18 in Richtung eines Pfeils 36 aus dem Aufnahmeelement 16 herausgezogen werden kann.

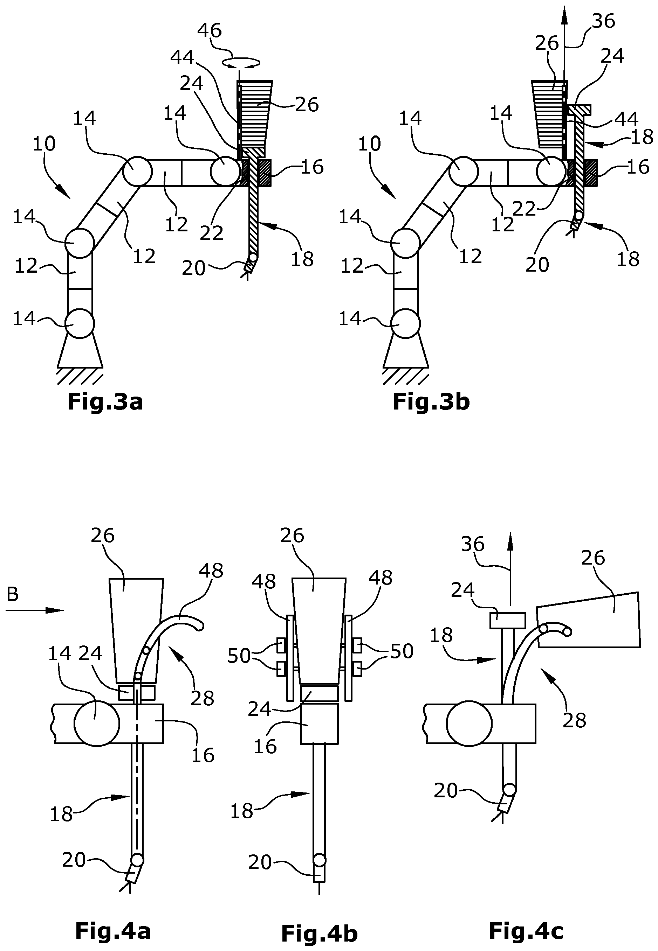

Bei der in Fig. 3a und 3b dargestellten Ausführungsform ist eine Schwenkachse 44 vorgesehen, die mit dem Aufnahmeelement 16 verbunden ist. Hierdurch ist ein Verschwenken der Antriebseinrichtung 26, wie durch den Pfeil 46 dargestellt, möglich. Zum Auswechseln des Instrumententrägers 18 (Fig. 3b) erfolgt ein Verschwenken der Antriebseinrichtung 26 um die Schwenkachse 44 in die in Fig. 3b dargestellte Position. Die Auswechslung des Instrumententrägers 18 erfolgt sodann wie vorstehend anhand beiden Ausführungsformen beschrieben.

Eine weitere bevorzugte Ausführungsform ist in Fig.4a bis 4c dargestellt. Diese Ausführungsform weist als Halteeinrichtung 28 einander gegenüberliegende Führungsschienen 48 auf. In den beiden Führungsschienen 48 sind jeweils zwei mit der Antriebseinrichtung 26 verbundene Führungszapfen 50 angeordnet. Aufgrund der gekrümmten Ausgestaltung der beiden Führungsschienen zwei mit der Antriebseinrichtung 26 verbundene Führungszapfen 50 angeordnet. Aufgrund der gekrümmten Ausgestaltung der beiden Führungsschienen 48 ist es möglich, die Antriebseinrichtung aus der Antriebsposition (Fig. 4a und 4b) in die Auswechselposition (Fig. 4c) überzuführen. Es ist sodann, wie vorstehend beschrieben wiederum möglich, den Instrumententräger 18 auszuwechseln.

Bei sämtlichen beschriebenen Ausführungsformen ist es möglich, zusätzliche Fixierelemente oder Dämpfungselemente vorzusehen, wie anhand der in Fig. 1a und 1b dargestellten Ausführungsform beschrieben.

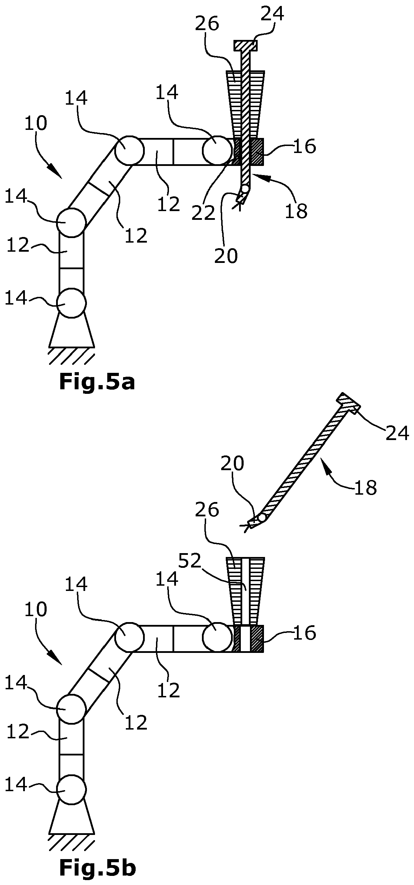

Bei einer weiteren exemplarischen Ausführungsform (Fig. 5a und 5b), , sind ähnliche und identische Bauteile wiederum mit denselben Bezugszeichen gekennzeichnet. In dieser Ausführungsform erfolgt kein Verändern der Position der Antriebseinrichtung 26. Vielmehr ist diese fest mit dem Aufnahmeelement 16 verbunden. Bei dieser Ausführungsform ist erfindungsgemäß eine Ausnehmung bzw. Einstecköffnung 52 vorgesehen. Diese erstreckt sich in Längsrichtung durch die gesamte Antriebseinrichtung 26 und fluchtet mit der in dem Aufnahmeelement 16 vorgesehenen Öffnung. Das Auswechseln des Instrumententrägers 18 ist somit durch ein einfaches Einstecken bzw. Herausziehen aus der Einstecköffnung 52 möglich.

## Patentansprüche

1. Halteeinrichtung für chirurgische Instrumente, insbesondere für die minimal-invasive Chirurgie, mit
einem Haltearm (10),
einem von dem Haltearm (10) getragenen Instrumententräger (18), der an seinem distalen Ende ein chirurgisches Instrument (20) trägt und einer mit dem Instrumententräger (18) verbundenen Antriebseinrichtung (26) zum Betätigen des Instruments (20), wobei die Antriebseinrichtung (26) über eine Halteeinrichtung (28), die ein Schwenkelement (30, 40) aufweist, derart mit dem Haltearm (10) verbunden ist, dass die gesamte Antriebseinrichtung (26) aus einer Antriebsposition in eine Auswechselposition verschwenkbar oder verdrehbar ist und
der Instrumententräger in der Arbeitsposition unmittelbar mit der Antriebseinrichtung verbunden ist.

2. Halteeinrichtung nach Anspruch 1, **dadurch gekennzeichnet, dass** in der Antriebsposition die Antriebseinrichtung (26) mit dem Instrumententräger (18) zum Betätigen des chirurgischen Instruments (20) verbunden ist.

3. Halteeinrichtung nach Anspruch 1 oder 2, **dadurch gekennzeichnet, dass** in der Auswechselposition die Antriebseinrichtung (26) vom Instrumententräger (18) zum Auswechseln des Instrumententrägers (18) entkoppelt ist.

4. Halteeinrichtung nach einem der Ansprüche 1 bis 3, **dadurch gekennzeichnet, dass** in der Auswechselposition die Antriebseinrichtung (26) mit dem Haltearm (20) verbunden bleibt.

5. Halteeinrichtung nach einem der Ansprüche 1 bis 4, **dadurch gekennzeichnet, dass** die Halteeinrichtung (28) ein Schiebeelement (48,50), insbesondere eine Führungsschiene (48) zum Verschieben der Antriebseinrichtung (26) zwischen der Antriebsposition und der Auswechselposition aufweist.

6. Halteeinrichtung nach einem der Ansprüche 1 bis 5, **dadurch gekennzeichnet, dass** die Halteeinrichtung (28) mindestens ein Fixierelement zum Fixieren der Antriebseinrichtung (26) in der Antriebsposition und/oder der Auswechselposition aufweist.

7. Halteeinrichtung nach einem der Ansprüche 1 bis 6, **dadurch gekennzeichnet, dass** der Instrumententräger (18) in der Antriebsposition unmittelbar mit der Antriebseinrichtung (26) über Steckkontakte verbunden ist.

8. Halteeinrichtung nach einem der Ansprüche 1 bis 7, **dadurch gekennzeichnet, dass** mit der Halteeinrichtung (28) eine Zusatz-Antriebseinrichtung verbindbar ist.

9. Halteeinrichtung nach einem der Ansprüche 1 bis 8, **dadurch gekennzeichnet, dass** die Halteeinrichtung (28) mindestens ein Dämpfungselement zum Dämpfen der Bewegung der Antriebseinrichtung (26) in die Antriebsposition und/oder in die Auswechselposition aufweist.

10. Verfahren zum Auswechseln eines chirurgischen Instruments, insbesondere eines Instruments für die minimal-invasive Chirurgie, an einer Halteeinrichtung nach einem der Ansprüche 1 bis 9, bei welchem die Antriebseinheit (26) aus einer Antriebsposition in eine Auswechselposition überführt wird, wobei die Antriebseinrichtung (26) in der Auswechselposition mit dem Haltearm (10) verbunden bleibt und der Instrumententräger in der Arbeitsposition unmittelbar mit der Antriebseinrichtung verbunden ist.

11. Verfahren nach Anspruch 10, bei welchem zum und/oder beim Überführen der Antriebseinrichtung (26) in die Auswechselposition die Antriebseinrichtung (26) von dem Instrumententräger (18) getrennt wird.

12. Verfahren nach Anspruch 10 oder 11, bei welchem der Instrumententräger (18) in der Auswechselposition ausgewechselt wird, ohne die Antriebseinrichtung (26) vom Haltearm (10) zu trennen.

## Claims

1. A holding device for surgical instruments, in particular for the minimally invasive surgery, comprising
a holding arm (10),
an instrument carrier (18) held by the holding arm (10), which instrument carrier carries a surgical instrument (20) at its distal end, and
a driving device (26) connected to the instrument carrier (18) for operating the instrument (20),
wherein the driving device (26) is connected to the holding arm (10) via a holding device (28) comprising a pivoting element (30, 40) such that the entire driving device (26) is adapted to be pivoted or rotated from a driving position into an exchanging position, and
the instrument carrier, in the operating position, is directly connected to the driving device.

2. The holding device according to claim 1, **characterized in that** in the driving position the driving device (26) is connected to the instrument carrier (18) for operating the surgical instrument (20).

3. The holding device according to claim 1 or 2, **characterized in that** in the exchanging position the driving device (26) is decoupled from the instrument carrier (18) for exchanging the instrument carrier (18).

4. The holding device according to any one of claims 1 to 3, **characterized in that** in the exchanging position the driving device (26) remains connected to the holding arm (20).

5. The holding device according to any one of claims 1 to 4, **characterized in that** the holding device (28) comprises a sliding element (48, 50), in particular a guide rail (48), for displacing the driving device (26) between the driving position and the exchanging position.

6. The holding device according to any one of claims 1 to 5, **characterized in that** the holding device (28) comprises at least one fixing element for fixing the driving device (26) in the driving position and/or the exchanging position.

7. The holding device according to any one of claims 1 to 6, **characterized in that** the instrument carrier (18), in the driving position, is directly connected to the driving device (26) via plug contacts.

8. The holding device according to any one of claims 1 to 7, **characterized in that** an additional driving device is adapted to be connected to the holding device (28).

9. The holding device according to any one of claims 1 to 8, **characterized in that** the holding device (28) comprises at least one damping element for damping the movement of the driving device (26) into the driving position and/or into the exchanging position.

10. A method for exchanging a surgical instrument, in particular an instrument for the minimally invasive surgery, at a holding device according to any one of claims 1 to 9, where the driving device (26) is moved from a driving position into an exchanging position, wherein the driving device (26), in the exchanging position, remains connected to the holding arm (10), and
the instrument carrier, in the operating position, is directly connected to the driving device.

11. The method according to claim 10, wherein the driving device (26) is detached from the instrument carrier (18) for and/or during moving the driving device (26) into the exchanging position.

12. The method according to claim 10 or 11, wherein the instrument carrier (18), in the exchanging position, is exchanged without detaching the driving device (26) form the holding arm (10).

## Revendications

1. Dispositif de maintien pour instruments chirurgicaux, en particulier pour chirurgie faiblement invasive, comprenant
un bras de maintien (10),
un porte-instrument (18) porté par le bras de maintien (10), portant un instrument chirurgical (20) à son extrémité distale et
un dispositif d'entraînement (26) raccordé au porte-instrument (18) pour actionner l'instrument (20),
dans lequel le dispositif d'entraînement (26) est relié au bras de maintien (10) par le biais d'un dispositif de maintien (28) comportant un élément de pivot (30, 40) de telle sorte que l'ensemble du dispositif d'entraînement (26) peut pivoter ou tourner depuis une position d'entraînement vers une position de remplacement et
le porte-instrument est, dans la position de travail, directement relié au dispositif d'entraînement.

2. Dispositif de maintien selon la revendication 1, **caractérisé en ce que** dans la position d'entraînement le dispositif d'entraînement (26) est relié au porte-instrument (18) afin d'actionner l'instrument chirurgical (20) .

3. Dispositif de maintien selon la revendication 1 ou 2, **caractérisé en ce que** dans la position de remplacement le dispositif d'entraînement (26) est découplé du porte-instrument (18) afin de remplacer le porte-instrument (18) .

4. Dispositif de maintien selon l'une des revendications 1 à 3, **caractérisé en ce que** dans la position de remplacement le dispositif d'entraînement (26) reste relié au bras de maintien (20).

5. Dispositif de maintien selon l'une des revendications 1 à 4, **caractérisé en ce que** le dispositif de maintien (28) comporte un élément coulissant (48, 50), en particulier un rail de guidage (48) pour faire coulisser le dispositif d'entraînement (26) entre la position d'entraînement et la position de remplacement.

6. Dispositif de maintien selon l'une des revendications 1 à 5, **caractérisé en ce que** le dispositif de maintien (28) comporte au moins un élément de blocage afin de bloquer le dispositif d'entraînement (26) dans la position d'entraînement et/ou dans la position de remplacement.

7. Dispositif de maintien selon l'une des revendications 1 à 6, **caractérisé en ce que** le porte-instrument (18) est directement relié au dispositif d'entraînement (26) par le biais de contacts enfichables dans la position d'entraînement.

8. Dispositif de maintien selon l'une des revendications 1 à 7, **caractérisé en ce qu'**un dispositif d'entraînement additionnel peut être relié au dispositif de maintien (28) .

9. Dispositif de maintien selon l'une des revendications 1 à 8, **caractérisé en ce que** le dispositif de maintien (28) comporte au moins un élément amortisseur afin d'amortir le mouvement du dispositif d'entraînement (26) vers la position d'entraînement et/ou vers la position de remplacement.

10. Procédé de remplacement d'un instrument chirurgical, en particulier d'un instrument pour chirurgie faiblement invasive, sur un dispositif de maintien selon l'une des revendications 1 à 9, lors duquel l'unité d'entraînement (26) est passée d'une position d'entraînement à une position de remplacement, dans lequel le dispositif d'entraînement (26) reste relié au bras de maintien (10) dans la position de remplacement et le porte-instrument est, dans la position de travail, directement relié au dispositif d'entraînement.

11. Procédé selon la revendication 10, lors duquel le dispositif d'entraînement (26) est séparé du porte-instrument (18) afin de passer le dispositif d'entraînement (26) dans la position de remplacement et/ou lors de ce passage.

12. Procédé selon la revendication 10 ou 11, lors duquel le porte-instrument (18) est remplacé dans la position de remplacement, sans séparer le dispositif d'entraînement (26) du bras de maintien (10).
